# EUROPEAN PATENT APPLICATION

(11) **EP 4 814 478 A1**
(43) Date of publication of application: **30.09.2026**
(21) Application number: 24893092.7
(22) Date of filing: 17.10.2024
(51) Int. Cl.: G01N 21/65, G16B 40/00

(54) **CELL CULTURE SOLUTION METABOLITE CONCENTRATION DETERMINATION METHOD AND APPARATUS, DEVICE AND MEDIUM**

(30) Priority: 20.11.2023 CN 202311548419
(71) Applicant: Wuxi Biologics Co., Ltd., Wuxi, Jiangsu 214000 (CN)
(72) Inventor: HUANG, Yanting, Wuxi, Jiangsu 214000 (CN); WANG, Lijun, Wuxi, Jiangsu 214000 (CN); TANG, Hao, Wuxi, Jiangsu 214000 (CN); XIANG, Shaoxun, Wuxi, Jiangsu 214000 (CN); WANG, Zhaoyang, Wuxi, Jiangsu 214000 (CN); CHEN, Tao, Wuxi, Jiangsu 214000 (CN)
(74) Representative: Santarelli
(86) International application number: PCT/CN2024/125407
(87) International publication number: WO 2025/107933

(57) **Abstract**

A cell culture solution metabolite concentration determination method and apparatus, a computer device and a computer-readable storage medium. The method includes: acquiring spectral data of a detection-target cell culture solution at a target time point and a preset cell culture environmental parameter, the spectral data having a preset length; and inputting the spectral data and the preset cell culture environmental parameter into a target concentration prediction model to obtain a corresponding metabolite concentration prediction result of the detection-target cell culture solution at the target time point. The target concentration prediction model includes a plurality of data feature extraction modules, and the plurality of data feature extraction modules are respectively configured to perform feature extraction on the spectral data and the preset cell culture environmental parameter along different feature analysis dimensions. The apparatus corresponding to the method includes a data acquisition unit and a concentration prediction unit. The computer device and the computer-readable storage medium include a processor for executing the method, and a computer program. Data feature analysis is performed based on multi-modal data of the cell culture solution under test, such that a more accurate concentration prediction result of the cell culture solution can be obtained.

## Description

### CROSS-REFERENCE TO RELATED APPLICATIONS

This application is based on and claims the priority to the CN application No. 202311548419.6, filed on November 20, 2023, the disclosure of which is incorporated herein by reference in its entirety.

### TECHNICAL FIELD

The present disclosure relates to the field of biological detection technologies, and in particular to a method, an apparatus, a device and a medium for determining a concentration of a metabolite in a cell culture solution.

### BACKGROUND

In the research and application of biotechnology, many scenarios require monitoring concentrations of cell metabolites in a cell culture solution during cell culture. Raman spectroscopy has advantages such as being non-destructive, highly sensitive, and label-free, and is gradually being applied in detection of metabolites in the cell culture.

### SUMMARY

In a first aspect, the present disclosure provides a method for determining a concentration of a metabolite in a cell culture solution, the method including:
acquiring spectral data of a detection-target cell culture solution at a target time point and a preset cell culture environmental parameter, the spectral data having a preset length;
inputting the spectral data and the preset cell culture environmental parameter into a target concentration prediction model to obtain a corresponding metabolite concentration prediction result of the detection-target cell culture solution at the target time point,
where the target concentration prediction model includes a plurality of data feature extraction modules, and the plurality of data feature extraction modules are respectively configured to perform feature extraction on the spectral data and the preset cell culture environmental parameter along different feature analysis dimensions.

In a second aspect, the present disclosure provides an apparatus for determining a concentration of a metabolite in a cell culture solution, the apparatus including:
a data acquisition unit configured to acquire spectral data of a detection-target cell culture solution at a target time point and a preset cell culture environmental parameter, the spectral data having a preset length;
a concentration prediction unit configured to input the spectral data and the preset cell culture environmental parameter into a target concentration prediction model to obtain a corresponding metabolite concentration prediction result of the detection-target cell culture solution at the target time point,
where the target concentration prediction model includes a plurality of data feature extraction modules, and the plurality of data feature extraction modules are respectively configured to perform feature extraction on the spectral data and the preset cell culture environmental parameter along different feature analysis dimensions.

In a third aspect, the present disclosure further provides a computer device, the computer device including:
one or more processors; and
a memory configured to store one or more programs,
where the one or more programs, when executed by the one or more processors, cause the one or more processors to implement the method for determining the concentration of the metabolite in the cell culture solution as provided in any embodiment of the present disclosure.

In a fourth aspect, the present disclosure further provides a non-transitory computer-readable storage medium having a computer program stored thereon which, when executed by a processor, implements the method for determining the concentration of the metabolite in the cell culture solution as provided in any embodiment of the present disclosure.

### BRIEF DESCRIPTION OF THE DRAWINGS

FIG. 1 is a flowchart of a method for determining a concentration of a metabolite in a cell culture solution according to some embodiments of the present disclosure;
FIG. 2 is a flowchart of a method for determining a concentration of a metabolite in a cell culture solution according to some embodiments of the present disclosure;
FIG. 3 is a schematic diagram of a training process of a target concentration prediction model according to some embodiments of the present disclosure;
FIG. 4 is a trend chart of components in tank 6 during an experiment according to some embodiments of the present disclosure;
FIG. 5 is a trend chart of components in tank 9 during an experiment according to some embodiments of the present disclosure;
FIG. 6 is a flowchart of a method for determining a concentration of a metabolite in a cell culture solution according to some embodiments of the present disclosure;
FIG. 7 is a schematic diagram of an application instance of a target concentration prediction model according to some embodiments of the present disclosure;
FIG. 8 is a schematic diagram of automatically controlled glucose concentration matched with a peristaltic pump according to some embodiments of the present disclosure;
FIG. 9 is a schematic diagram of manually controlled glucose concentration matched with a peristaltic pump according to some embodiments of the present disclosure;
FIG. 10 is a schematic structural diagram of an apparatus for determining a concentration of a metabolite in a cell culture solution according to some embodiments of the present disclosure;
FIG. 11 is a schematic structural diagram of a computer device according to some embodiments of the present disclosure..

### DETAILED DESCRIPTION

The present disclosure will be described below in further detail with reference to the accompanying drawings and embodiments. It can be understood that the specific embodiments described herein are merely for explaining the present disclosure and are not intended to limit the present disclosure. It should also be noted that, for ease of description, the accompanying drawings show only the parts relevant to the present disclosure, not the entire structure.

In the process of implementing the present disclosure, it is found that due to the high-dimensional complexity of Raman spectroscopy data and diversity among the data, certain limitations exist in using simple unimodal machine learning or deep learning (such as text convolutional models) for metabolite concentration prediction, which results in a large prediction error.

The present disclosure provides a method, an apparatus, a device, and a medium for determining a concentration of a metabolite in a cell culture solution, which can be used for performing data feature analysis based on multimodal data of the cell culture solution under test, thereby obtaining a more accurate concentration prediction result for the cell culture solution.

Specifically, in the present disclosure, by acquiring preset-length spectral data of a detection-target cell culture solution at a target time point and a preset cell culture environmental parameter, and by inputting the spectral data and the preset cell culture environmental parameter into a target concentration prediction model, a corresponding metabolite concentration prediction result of the detection-target cell culture solution at the target time point is obtained. The target concentration prediction model includes a plurality of data feature extraction modules, and the plurality of data feature extraction modules are respectively configured to perform feature extraction on the spectral data and the preset cell culture environmental parameter along different feature analysis dimensions. The technical solutions of embodiments of the present disclosure solve the problem of inaccurate prediction results of existing concentration analysis models for cell culture solutions, and can perform data feature analysis based on multimodal data of the cell culture solution under test to obtain a more accurate concentration prediction result for the cell culture solution.

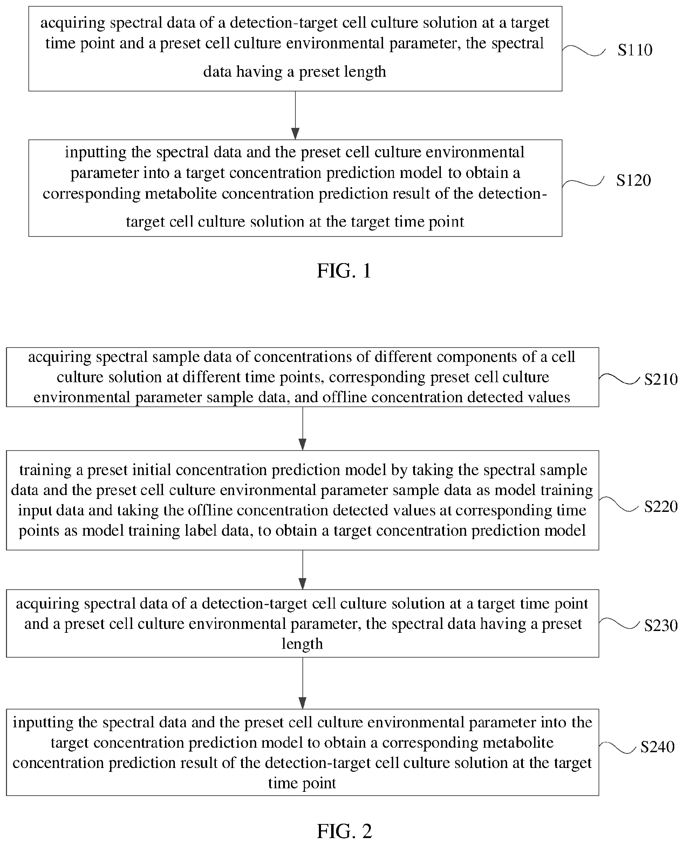

FIG. 1 is a flowchart of a method for determining a concentration of a metabolite in a cell culture solution according to some embodiments of the present disclosure. This embodiment is applicable to scenarios of biological experiments, especially cases where concentrations of cell metabolites are predicted during cell culture in a bioreactor. This method may be performed by an apparatus for determining a concentration of a metabolite in a cell culture solution, which may be implemented by software and/or hardware and integrated into a computer device with application development functions.

As shown in FIG. 1, in this embodiment, the method for determining the concentration of the metabolite in the cell culture solution includes the following steps S110 and S120.

At step S110, preset-length spectral data of a detection-target cell culture solution at a target time point and a preset cell culture environmental parameter are acquired.

Specifically, the detection-target cell culture solution may be a cell culture solution in which concentrations of cell metabolites need to be analyzed in any biological reaction process. The target time point may be any time point in the biological reaction process of the detection-target cell culture solution.

The spectral data may be any type of spectral information that can reflect a difference in concentrations between different substances in the cell culture solution. For example, the spectral data may be Raman spectral data. Online spectral data of the detection-target cell culture solution at the target time point may be obtained by using a Raman spectrometer. The preset length of the spectral data may be a superior data paradigm determined through experiments during the training process of the target concentration prediction model, or may be determined based on the experience of relevant technical personnel.

In the past, metabolite concentration detection in a cell culture solution typically relies solely on spectral data for concentration analysis, to obtain a metabolite concentration prediction result. However, in a biological reaction process, since an environmental parameter during cell culture may also affect the biological reaction process to some extent, the environmental parameter is also considered in the metabolite concentration analysis in this embodiment for metabolite concentration prediction, in order to obtain a more accurate prediction result. The concentration of the metabolite in the detection-target cell culture solution may be analyzed based on multimodal data of different dimensions obtained through different detection methods for the detection-target cell culture solution.

Specifically, the preset cell culture environmental parameter may include, but is not limited to, discrete metadata related to cell culture experiments such as cell line, clone, culture process, and the like, e.g., cell strain type, culture medium type, temperature and pH during cell culture and other parameters. The preset cell culture environmental parameter may be a combination of one or more of the above parameters. In some embodiments, the preset cell culture environmental parameter includes at least one of the following parameters: a cell line parameter, a cell clone parameter, a culture process parameter, or parameters during cell culture.

At step S120, the spectral data and the preset cell culture environmental parameter are input into a target concentration prediction model to obtain a corresponding metabolite concentration prediction result of the detection-target cell culture solution at the target time point.

Specifically, the target concentration prediction model may be a pre-trained neural network model that can predict concentrations of metabolites in the cell culture solution based on the input data and output the corresponding metabolite concentration prediction result. The reason why the target concentration prediction model can predict the concentrations of the metabolites in the cell culture solution more accurately is that, the target concentration prediction model may include a plurality of data feature extraction modules which are respectively configured to perform feature extraction on the spectral data and the preset cell culture environmental parameter along different feature analysis dimensions.

In addition, in some embodiments, the target concentration prediction model further includes a feature fusion module and a concentration prediction module. The feature fusion module is configured to perform feature fusion on features extracted by the plurality of data feature extraction modules respectively to obtain a target fusion feature. The concentration prediction module is configured to analyze the target fusion feature to obtain the metabolite concentration prediction result.

In some embodiments, the target concentration prediction model is determined by training a model having the above structure with: the online spectral data of concentrations of different components changing over time obtained from the cell culture solution of the bioreactor using the Raman spectrometer, collected corresponding culture environmental parameter values, and offline target values for respective batches obtained by taking samples at regular intervals during the experiment and using offline detection devices. In this process, for example, the data feature extraction module and the feature fusion module designed for different data characteristics may be combined to provide rich contextual information for the concentration prediction module of the entire model, thereby improving the prediction and analysis accuracy of the target concentration prediction model.

Therefore, by inputting the acquired spectral data and preset cell culture environmental parameter into the target concentration prediction model, the corresponding metabolite concentration prediction result of the detection-target cell culture solution at the target time point can be obtained.

In some embodiments, the data feature extraction modules in the target concentration prediction model include a first data feature extraction module, a second data feature extraction module, and a third data feature extraction module. The first data feature extraction module is configured to perform feature encoding analysis on the preset cell culture environmental parameter to obtain a target environmental parameter feature. The second data feature extraction module is configured to extract, from the spectral data, a spectral feature between spectral data of different frequency bands. The third data feature extraction module is configured to extract at least one of the following spectral features of the spectral data: a preset spectral statistics feature, a preset spectral feature, or a preset spectral peak analysis feature.

It should be noted that, the reason of incorporating the preset spectral statistics (such as mean, median, etc.), the preset spectral feature (such as spectral energy, spectral entropy, etc.), and the preset spectral peak analysis feature (such as number of peaks, average peak height, distance between peaks, etc.) into the algorithm is that, there are not only large differences in optical signals between Raman devices, but also differences within the same device (different degrees of drift exist in use). Differences between devices often require the development of a set of optical physics and mathematical strategies for conversion based on optical signal data, which is time-consuming and labor-intensive, and lacks accuracy. The current approach to address the differences between different channels within the device is to calibrate the optical fiber, optical coupling sensor, and laser source by using optical standards to bring the different optical channels to the same level. However, performing this approach during experiments may introduce unnecessary culture risks, such as bacterial contamination. In contrast, the embodiments of the present disclosure can effectively eliminate spectral differences between devices and within the same device by incorporating these statistics, thereby making the prediction results more accurate.

In some embodiments, the second data feature extraction module may be configured to extract, from the spectral data, the spectral feature between the spectral data of different frequency bands by using a neural network module with a self-attention mechanism. The self-attention mechanism may be introduced during the feature processing on the spectral data. In the Transformer model, the self-attention mechanism is a core component that can improve the model's parallel processing capabilities and understanding depth.

The self-attention mechanism is adopted based on a similarity between characteristics of Raman spectral data and natural language data. Firstly, a correlation between spectral intensities of different frequency bands in the Raman spectrum may be compared to semantic and grammatical relationships between words or phrases in natural language. In the Raman spectrum, the different frequency bands may reflect characteristics of the same or similar chemical compositions, and therefore there may be an inherent correlation therebetween. Similarly, in a text of natural language, different words or phrases may have semantic dependencies and influence to each other. Secondly, the analysis of the Raman spectrum and the analysis of the natural language data both require capturing these complex internal relationships. The method based on the self-attention mechanism emphasizes precisely this point, which can capture the correlation between the different frequency bands in the Raman spectrum by dynamically assigning weights to different parts, and this characteristic is analogous to the self-attention mechanism's ability to handle long-distance dependencies in natural language. Furthermore, neural network models based on self-attention mechanisms can effectively overcome the distance limitations of spectral bands and effectively capture the correlation between metabolites and spectral values of various bands. This summarization of regular patterns can be done automatically without relying on manual feature selection or other steps, which improves the efficiency of the modeling and reasoning process to a certain extent. Finally, the structures of both Raman spectrum and natural language data can be represented as sequences to some extent. Because of this similarity, the self-attention mechanism can bridge these two fields, providing a unified and efficient method for quantitative analysis of Raman spectroscopy and deep understanding of natural language. The target concentration prediction model trained based on the above comprehensive data features has been verified by actual results to indeed obtain more accurate prediction results.

In the technical solution of this embodiment, by acquiring preset-length spectral data of a detection-target cell culture solution at a target time point and a preset cell culture environmental parameter, and by inputting the spectral data and the preset cell culture environmental parameter into a target concentration prediction model, a corresponding metabolite concentration prediction result of the detection-target cell culture solution at the target time point is obtained. The target concentration prediction model includes a plurality of data feature extraction modules, and the plurality of data feature extraction modules are respectively configured to perform feature extraction on the spectral data and the preset cell culture environmental parameter along different feature analysis dimensions. The technical solution of the embodiment of the present disclosure solves the problem of inaccurate prediction results of existing concentration analysis models for cell culture solutions, and can perform data feature analysis based on multimodal data of the cell culture solution under test to obtain more accurate concentration prediction results for the cell culture solution. In particular, by analyzing the preset-length spectral data of the detection-target cell culture solution at the target time point based on the self-attention mechanism, data features in a deeper level can be captured to reflect the final prediction result.

FIG. 2 is a flowchart of a method for determining a concentration of a metabolite in a cell culture solution according to some embodiments of the present disclosure. This embodiment has the same inventive concept as the method for determining the concentration of the metabolite in the cell culture solution in the above embodiment, and further describes the process of training the target concentration prediction model. This method may be performed by an apparatus for determining a concentration of a metabolite in a cell culture solution, which may be implemented by software and/or hardware and integrated into a computer device with application development functions.

As shown in FIG. 2, the method for determining the concentration of the metabolite in the cell culture solution in this embodiment includes the following steps S210 to S240.

At step S210, spectral sample data of concentrations of different components of a cell culture solution at different time points, corresponding preset cell culture environmental parameter sample data, and offline concentration detected values are obtained.

For example, the process of obtaining the target concentration prediction model in the above embodiment may be roughly divided into following stages: data processing, model building, data training, and model validation.

In this step, data processing is performed first to construct sample data for model training.

Specifically, the online spectral data of concentrations of different components of the cell culture solution changing over time may be obtained by using, for example, the Raman spectrometer from the cell culture solution in the bioreactor, and culture environmental parameter values are collected, while obtaining offline target values of the concentrations of the different components of the cell culture solution by taking samples at regular intervals during the experiment and using offline detection devices.

For example, the spectral data is obtained by mounting a probe of a pre-set Raman spectrometer into the bioreactor and directly immersing it into the cell culture solution. Raman spectra of different bioreactors are recorded throughout the experiment. For a single recorded spectrum, 30 subsequent spectra may be captured with a 10-second exposure time and averaged, and the acquisition interval for each bioreactor is approximately 5 minutes. The laser excitation wavelength during the operation of the spectrometer is 785 nm, providing spectral coverage (Raman shift) of 100-3425 cm⁻¹.

For example, the preset cell culture environmental parameter sample data may include: conditions such as temperature, shaker speed, carbon dioxide concentration, and culture medium parameters when cells are cultured in a shaker during a seed stage; reactor size (e.g., 3L and 250L) used in a production and cultivation stage; and initial culture volume, culture temperature, pH set value, dissolved oxygen saturation, initial inoculation density, and supplemented culture medium parameters, and the like, of the cell culture solution.

The offline concentration detected values of the different components may be target values detected upon sampling five times a day at time intervals, such as, but not limited to, viable cell density (VCD), cell viability, and average cell diameter (AvgDiam) detected by a cell counter (such as Vi-CELL XR); glucose, lactate, ammonium ion (NH4⁺), glutamate, glutamine, iron ion, phosphate ion, lactate dehydrogenase (LDH), and target protein concentration (titer) determined by a biochemical analyzer (such as Cedex Bio HT); osmolality detected by an osmometer (such as OsmoPRO); and acidity (pH), partial pressure of carbon dioxide (pCO₂), sodium ion (Na⁺), and potassium ion (K⁺) detected by a blood gas analyzer.

At step S220, a preset initial concentration prediction model is trained by taking the spectral sample data and the preset cell culture environmental parameter sample data as model training input data and taking the offline concentration detected values at corresponding time points as model training label data, to obtain the target concentration prediction model.

In each model training sample, the spectral sample data and the preset cell culture environmental parameter sample data are taken as the model training input data, and the offline concentration detected value at the corresponding time point is taken as the model training label data. The time points at which the spectra are detected and the time points at which the offline concentration detected values are detected may be in one-to-one correspondence.

In order to analyze the acquired multimodal data, when constructing the model, the preset initial concentration prediction model includes a plurality of data feature extraction modules configured to extract data features along different dimensions to obtain higher-order data features. In some embodiments, the data feature extraction modules include a first data feature extraction module, a second data feature extraction module, and a third data feature extraction module. The first data feature extraction module is configured to perform feature encoding analysis on the preset cell culture environmental parameter to obtain a target environmental parameter feature. The second data feature extraction module is configured to extract, from the spectral data, a spectral feature between spectral data of different frequency bands. The third data feature extraction module is configured to extract at least one of the following spectral features of the spectral data: a preset spectral statistics feature, a preset spectral feature, or a preset spectral peak analysis feature. In some examples, the second data feature extraction module is a neural network module with a self-attention mechanism, which can extract, from the spectral data, the spectral feature between the spectral data of different frequency bands. The main idea of the attention mechanism is to make the model pay more attention to important inputs by assigning different weights to input signals at different positions when processing sequential data. In deep learning models, the attention mechanism is typically implemented by adding additional network layers, and these layers can learn how to compute the weights and apply the weights to the input signals.

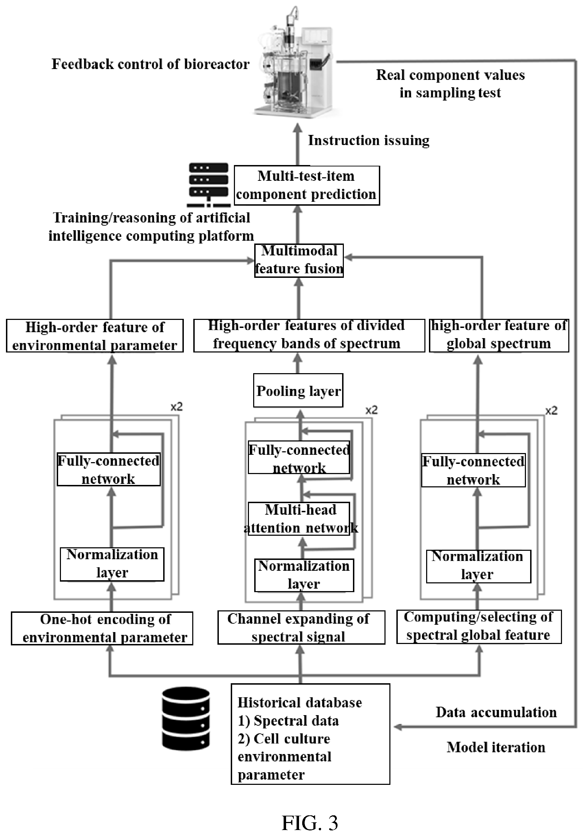

For example, the process as shown in FIG. 3 may be referred to for the process of training the preset initial concentration prediction model. In FIG. 3, data in a historical database is the sample data for model training which is input into the model and subjected to feature extraction through three paths (corresponding to the three data feature extraction modules) respectively. In this process, after one-hot encoding is performed on the preset cell culture environmental parameter sample data, a high-order feature of the environmental parameter is obtained through a dual-channel normalization layer and fully-connected network, which corresponds to the first data feature extraction module. Channel expanding is performed on the spectral signal, and high-order features of divided frequency bands of the spectrum can be obtained through a dual-channel normalization layer, multi-head attention network and fully-connected network, and a pooling process, which correspond to the second data feature extraction module. In addition, a global feature of the spectral data is calculated and selected, and a high-order feature of the global spectrum is obtained through the dual-channel normalization layer and fully-connected network, which corresponds to the third data feature extraction module.

The multi-head attention is an extended form of the attention mechanism, and can extract information more effectively when processing sequential data. In the multi-head attention, multiple sets of attention weights are used, each set of weights can learn different semantic information, and a context vector will be generated for each set of weights. Finally, these context vectors are concatenated and then subjected to a linear transformation to obtain a final output.

Furthermore, after feature extraction performed on the spectral data and the cell culture environmental parameter along multiple dimensions as shown in FIG. 3, multimodal feature fusion is performed on the extracted high-order feature of the environmental parameter, the high-order features of the divided frequency bands of the spectrum, and the high-order feature of the global spectrum. A fused result is then used by the concentration prediction module to perform multi-test-item component prediction, that is, to predict the concentrations of the components of the cell culture solution, thus obtaining the final prediction result.

Furthermore, it should be noted that during the model training stage, in addition to the model structure as shown in FIG. 3 (attention-based multimodal Raman spectroscopy transformer model (RAFORMER-multimodal)), this embodiment further constructs various machine learning models, including Partial Least Squares Regression (PLSR), Gradient Boosting Decision Tress (GBDT), text convolutional model (TextCNN), and attention-based Raman spectroscopy transformer model in which only Raman spectral data is used (RAFORMER-unimodal). Furthermore, the constructed model structures are trained based on model training samples, and then validated, tested and compared. Specifically, firstly, the validation results of the test set are used to ensure that each trained model has stable inference capabilities. Then, based on the stable performance of the test set, wet experiments are conducted to evaluate the performance of all models in order to ensure that the models are usable in production. The procedure of the wet experiments is described as follows: under the same culture conditions as the historical training data, multiple batches of cell culture experiments are tested, and Raman spectral data are collected by scanning the bioreactors; and data is collected at four time points every day and offline data is collected at the same time points upon taking samples, for one complete culture cycle. Furthermore, based on the acquired data, the model is evaluated. From the root mean square error values of the model predicted values and the offline measured values, the attention-based multimodal Raman spectroscopy transformer model shows higher accuracy and better performance; moreover, this model has not been tuned for a single application scenario through steps such as selection of hyperparameters, which greatly saves the effort required for manual intervention in the modeling process. This also enhances the versatility of RAFORMER and the possibility of its large-scale application in industrial production. Through analysis and comparation on various model structures, the superior performance of the RAFORMER-multimodal model may also be confirmed as it has considered environmental parameters related to cell culture experiments. Furthermore, based on the diversity of tested bioreactors in terms of culture processes, the multimodal model has greater potential to be used universally to a variety of clones and cell lines compared to the unimodal model, which can effectively overcome a limitation of the unimodal model that only emphasizes the resolving of the spectral data, thereby reducing the loss in prediction accuracy.

In addition, the variation trends predicted by the RAFORMER-multimodal model and the baseline models are compared with each other, and the Pearson correlation coefficient is used to evaluate the degree of fit of this trend. By comparing the performance of the RAFORMER-multimodal model with the performances of the two traditional models, GBDT and PLSR, on the above-mentioned different bioreactors, it is found that the RAFORMER-multimodal model shows an improvement in the Pearson correlation coefficient compared with GBDT and PLSR for all concentration test items.

In summary, it can be determined that the target concentration prediction model trained in this embodiment can obtain more accurate metabolite concentration prediction result of the cell solution based on the analysis on the multimodal data.

Specifically, the detailed process of evaluating the above models is as follows.

Concentrations of metabolites in cell culture solutions in two selected bioreactors are predicted and analyzed using the trained RAFORMER-multimodal model, the RAFORMER-unimodal model, the GBDT model, the TextCNN model, and the PLSR model. The two bioreactors are labeled as tank 6 and tank 9, respectively. Evaluation results of these two bioreactors are selected for presentation because the two bioreactors represent 3L and 200L culture processes respectively, as well as processes of enhanced fed-batch culture and traditional fed-batch culture respectively, which can comprehensively reflect the applicability of models under different conditions. For the evaluation comparison results of the tank 6 and the tank 9, please refer to Table 1 and Table 2 respectively.

**Table 1: Root mean square errors of model predicted values and offline measured values for the bioreactor tank 6**

| Process parameters | | RAFORMER-multimodal | GBDT | PLSR | | Unit | Parameter range |
|---|---|---|---|---|---|---|---|
| | VCD | 3.713315 | 9.701639 | 5.935412 | E6cell/mL | | 0.70 - 9.00 |
| Glucose | | 0.843273 | 1.380617 | 1.553841 | | g/L | 0.50 - 9.00 |
| | Iron | 37.43598 | 73.21201 | 73.89616 | | mg/L | 85 - 180 |
| Glutamine | | 0.546186 | 1.745281 | 0.940769 | | mM | 0.45 - 4.00 |
| | pH | 0.072274 | 0.175187 | 0.12444 | | | 6.58 - 7.27 |
| | pCO₂ | 6.738848 | 11.43872 | 8.539133 | mmHg | | 12.1 - 71.9 |
| | Na⁺ | 3.652582 | 6.236039 | 8.572321 | | mM | 88 - 133 |
| | K⁺ | 3.462971 | 3.694135 | 3.80827 | | mM | 3.85 - 8.10 |
| Lactate | | 0.22798 | 0.269963 | 0.198929 | | g/L | 0.15 - 2.45 |
| | NH4⁺ | 1.058867 | 1.811047 | 1.957382 | | mM | 0.45 - 9.00 |
| Glutamate | | 0.610238 | 2.664974 | 2.014847 | | mM | 2.70 - 15.00 |
| | OSMO | 21.04932 | 31.00926 | 31.21049 | mOsm/kg | | 260 - 450 |
| Viability | | 5.289168 | 8.086848 | 6.899572 | | % | 25 - 99.9 |
| AvgDiam | | 0.737902 | 2.86519 | 0.783846 | | um | 11.50 - 19.50 |
| | Titer | 0.928424 | 3.276577 | 2.882383 | | g/L | 0 - 6.0 |
| | LDH | 441.2631 | 1770.916 | 1993.386 | | U/L | 0 - 6000 |
| Phosphate | | 71.60987 | 90.70235 | 83.46153 | | mg/L | 350 - 1200 |

**Table 2: Root mean square errors of model predicted values and offline measured values for the bioreactor tank 9**

| Process parameters | RAFORMER-multimodal | GBDT | PLSR | Unit | Parameter range |
|---|---|---|---|---|---|
| VCD | 0.913781 | 1.880418 | 1.962796 | E6cell/mL | 0.70 - 49.00 |
| Glucose | 0.54204 | 1.012293 | 1.137349 | g/L | 0.50 - 9.00 |
| Iron | 2.273163 | 7.044774 | 6.938764 | mg/L | 85 - 180 |
| Glutamine | 0.452604 | 0.886814 | 0.87953 | mM | 0.45 - 4.00 |
| pH | 0.063159 | 0.080681 | 0.093612 | | 6.58 - 7.27 |
| pCO₂ | 12.78213 | 11.81791 | 12.53938 | mmHg | 12.1 - 71.9 |
| Na⁺ | 6.129993 | 4.648543 | 5.108044 | mM | 88 -133 |
| K⁺ | 0.728452 | 1.113267 | 1.098925 | mM | 3.85 - 18.10 |
| Lactate | 0.255035 | 0.224591 | 0.173402 | g/L | 0.15 - 2.45 |
| NH4⁺ | 0.586976 | 0.606469 | 0.726414 | mM | 0.45 - 9.00 |
| Glutamate | 0.429346 | 0.761154 | 0.643775 | mM | 2.70 - 15.00 |
| OSMO | 7.006372 | 28.54601 | 19.3163 | mOsm/kg | 260 - 450 |
| Viability | 7.032988 | 2.806012 | 5.726478 | % | 25 - 99.9 |
| AvgDiam | 0.689337 | 1.12204 | 1.820591 | um | 11.50 - 19.50 |
| Titer | 0.355118 | 0.591324 | 0.449733 | g/L | 0 - 6.0 |
| LDH | 657.2798 | 352.9957 | 253.5386 | U/L | 0 - 6000 |
| Phosphate | 34.93441 | 109.8995 | 89.16007 | mg/L | 350 - 1200 |

In these two tables, VCD represents viable cell density, Glucose represents glucose, Iron represents iron ion, Glutamine represents glutamine, pH represents acidity/alkalinity, pCO₂ represents partial pressure of carbon dioxide, Na⁺ represents sodium ion, K⁺ represents potassium ion, Lactate represents lactate, NH4⁺ represents ammonium ion, Glutamate represents glutamate, OSMO represents osmotic pressure, Viability represents cell viability, AvgDiam represents average cell diameter, Titer represents target protein concentration, LDH represents lactate dehydrogenase, and Phosphate represents phosphate ion.

By comparing the performance of the RAFORMER-multimodal model with performances of the two traditional models, GBDT and PLSR, on the two bioreactors, it can be determined that for all 17 test items, the RAFORMER-multimodal model improves the accuracy by 47.57% and 36.86% on average in terms of root mean square errors relative to GBDT and PLSR models, respectively, for the reactor tank 6, and by 14.67% and 17.47% for the reactor tank 9. Specifically, for each test item, it can be found that among the 17 test items, RAFORMER performs best in 16 test items for the reactor tank 6, and performs best in 12 test items for the reactor tank 9. The best performance refers to achieving the smallest root mean square error in comparisons between the RAFORMER-multimodal model and the baseline models, GBDT and PLSR. Another noteworthy point is that, compared to the baseline models, GBDT and PLSR, the RAFORMER-multimodal model has not been tuned for a single application scenario through steps such as selection of hyperparameters, which greatly saves the effort required for manual intervention in the modeling process. This also enhances the versatility of the RAFORMER-multimodal model and its potential for large-scale application in industrial production.

To verify the superiority of the multimodal model and ensure the fairness of the model performance comparison, the predictive performance of the RAFORMER-multimodal model and the predictive performance of the RAFORMER-unimodal model with only spectral data input are further compared for the two bioreactors, tank 6 and tank 9. For the evaluation comparison results of the tank 6 and the tank 9, please refer to Table 3 and Table 4 respectively:

**Table 3: Root mean square errors of model predicted values and offline measured values for the bioreactor tank 6**

| Process parameters | | RAFORMER-multimodal | | RAFORMER-unimodal | | Unit | | Parameter range |
|---|---|---|---|---|---|---|---|---|
| | VCD | | 3.713314701 | | 3.172992268 | E6cell/mL | | 0.70 - 49.00 |
| Glucose | | | 0.843273361 | | 0.957784274 | | g/L | 0.50 - 9.00 |
| | Iron | | 37.43597591 | | 60.44640728 | | mg/L | 85 - 180 |
| Glutamine | | | 0.546186403 | | 0.574262464 | | mM | 0.45 - 4.00 |
| | pH | | 0.072274445 | | 1.282868082 | | | 6.58 - 7.27 |
| pCO₂ | | | 6.738848485 | | 9.015574411 | mmHg | | 12.1 - 71.9 |
| | Na⁺ | | 3.652581943 | | 15.88855617 | | mM | 88 - 133 |
| | K⁺ | | 3.46297119 | | 3.577128264 | | mM | 3.85 - 18.10 |
| Lactate | | | 0.227980319 | | 0.300729799 | | g/L | 0.15 - 2.45 |
| NH4⁺ | | | 1.058867455 | | 1.137171702 | | mM | 0.45 - 9.00 |
| Glutamate | | | 0.61023846 | | 0.657835621 | | mM | 2.70 - 15.00 |
| OSMO | | | 21.04932497 | | 33.04471882 | mOsm/kg | | 260 - 450 |
| Viability | | | 5.28916793 | | 9.309222936 | | % | 25 - 99.9 |
| AvgDiam | | | 0.737902106 | | 2.284397101 | | um | 11.50 -19.50 |
| | Titer | | 0.928424497 | | 1.263533082 | | g/L | 0 - 6.0 |
| | LDH | | 441.2630814 | | 522.993953 | | U/L | 0 - 6000 |
| Phosphate | | | 71.60986569 | | 147.5284062 | | mg/L | 350 - 1200 |

**Table 4. Root mean square errors of model predicted values and offline measured values for the bioreactor tank 9**

| Process parameters | RAFORMER-multimodal | RAFORMER-unimodal | | Unit | Parameter range |
|---|---|---|---|---|---|
| VCD | 0.913780577 | 1.712740586 | E6cell/mL | | 0.70 -- 49.00 |
| Glucose | 0.54203976 | 0.862373849 | | g/L | 0.50 - 9.00 |
| Iron | 2.273162991 | 22.83208488 | mg/L | | 85 - 180 |
| Glutamine | 0.452603719 | 0.607982898 | | mM | 0.45 - 4.00 |
| pH | 0.063159219 | 1.257158596 | | | 6.58 - 7.27 |
| pCO₂ | 12.78212886 | 15.37680782 | mmHg | | 12.1 - 71.9 |
| Na⁺ | 6.12999264 | 12.65110701 | mM | | 88 - 133 |
| K⁺ | 0.72845242 | 1.092446034 | mM | | 3.85 - 18.10 |
| Lactate | 0.255034973 | 0.279460445 | | g/L | 0.15 - 2.45 |
| NH4⁺ | 0.586975846 | 0.528184514 | | mM | 0.45 - 9.00 |
| Glutamate | 0.429346213 | 1.320674032 | | mM | 2.70 - 15.00 |
| OSMO | 7.006372312 | 22.67719824 | mOsm/kg | | 260 - 450 |
| Viability | 7.032987838 | 8.659964682 | | % | 25 - 99.9 |
| AvgDiam | 0.689336691 | 2.726716687 | | um | 11.50 - 19.50 |
| Titer | 0.355118119 | 0.526046097 | g/L | | 0 - 6.0 |
| LDH | 657.2797856 | 556.4714381 | U/L | | 0 - 6000 |
| Phosphate | 34.93440647 | 122.9367997 | | mg/L | 350 - 1200 |

By comparing the performances of the RAFORMER-unimodal model and the RAFORMER-multimodal model for the two bioreactors, it can be found that for all 17 test items, the RAFORMER-multimodal model improves the accuracy by 30.40% on average in terms of root mean square errors relative to the RAFORMER-unimodal model for the reactor tank 6, and by 41.75% for the reactor tank 9. Specifically, for each test item, it can be found that among the 17 test items, the RAFORMER-multimodal model performs best in 16 test items for the reactor tank 6, and performs best in 15 test items for the reactor tank 9. The best performance refers to achieving the smallest root mean square error in the comparison between the RAFORMER-multimodal model and the RAFORMER-unimodal model.

This result strongly demonstrates the superior performance of the RAFORMER-multimodal model as it has considered environmental parameters related to cell culture experiments. Furthermore, based on the diversity of the two tested bioreactors in terms of culture processes, the RAFORMER-multimodal model has greater potential to be used universally to a variety of clones and cell lines compared to the unimodal model, which can effectively overcome a limitation of the unimodal model that only emphasizes the resolving of spectral data, thereby reducing the loss in prediction accuracy.

In addition to evaluating the root mean square errors of the models, the difference between the variation trends predicted by the RAFORMER-multimodal model and the baseline models is also evaluated, and the Pearson correlation coefficient is used to evaluate the degree of fit of this trend. Please refer to Tables 5 and 6 for detailed evaluation results.

**Table 5: Pearson correlation coefficients of model predicted values and offline measured values for the bioreactor tank 6**

| Process parameters | | RAFORMER-multimodal | GBDT | PLSR |
|---|---|---|---|---|
| | VCD | 0.905372297 | 0.502574985 | 0.553810369 |
| Glucose | | 0.953500414 | 0.736731995 | 0.92980928 |
| | Iron | 0.170242406 | -0.700499926 | -0.738249779 |
| Glutamine | | 0.944098713 | 0.715915947 | 0.931215158 |
| | pH | 0.846043273 | 0.604745873 | 0.599429936 |
| pCO2 | | 0.568536766 | -0.100024754 | 0.243373543 |
| | Na+ | 0.308478187 | 0.079462401 | -0.007145086 |
| | K+ | 0.593442311 | 0.552581153 | 0.592347291 |
| Lactate | | 0.955488716 | 0.881222719 | 0.946135467 |
| NH4+ | | 0.558043378 | 0.481936269 | 0.480796009 |
| Glutamate | | 0.83773165 | 0.396497076 | 0.660377411 |
| OSMO | | 0.531828217 | 0.1289012 | 0.041187009 |
| Viability | | 0.949010384 | 0.80936637 | 0.908831869 |
| AvgDiam | | 0.938260499 | -0.349261029 | 0.853397329 |
| | Titer | 0.989585427 | 0.910202719 | 0.959285842 |
| | LDH | 0.984040009 | 0.854746669 | 0.9211994 |
| Phosphate | | 0.912219423 | 0.72356352 | 0.885837973 |

**Table 6: Pearson correlation coefficients between model predicted values and offline measured values for the bioreactor tank 9**

| Process parameters | | RAFORMER | | GBDT | PLSR |
|---|---|---|---|---|---|
| | VCD | 0.974184 | | 0.884372 | 0.881397 |
| Glucose | | 0.957969 | | 0.458617 | 0.835644 |
| | Iron | 0.983576 | | 0.885184 | 0.953718 |
| Glutamine | | 0.976015 | | 0.939541 | 0.893836 |
| | pH | | 0.92558 | 0.799699 | 0.719813 |
| pCO₂ | | -0.45484 | | -0.26878 | -0.17165 |
| | Na⁺ | 0.820508 | | 0.850363 | 0.80105 |
| | K⁺ | 0.904431 | | 0.777484 | 0.853984 |
| Lactate | | 0.988182 | | 0.92142 | 0.976415 |
| NH4⁺ | | 0.963026 | | 0.90085 | 0.880874 |
| Glutamate | | 0.987507 | | 0.975352 | 0.973965 |
| OSMO | | 0.983426 | | 0.961691 | 0.973604 |
| Viability | | 0.97151 | | 0.97526 | 0.959102 |
| AvgDiam | | 0.909183 | | 0.783828 | 0.08637 |
| Titer | | 0.971853 | | 0.945887 | 0.983593 |
| LDH | | 0.99595 | | 0.954765 | 0.981075 |
| Phosphate | | 0.993444 | | 0.936647 | 0.972697 |

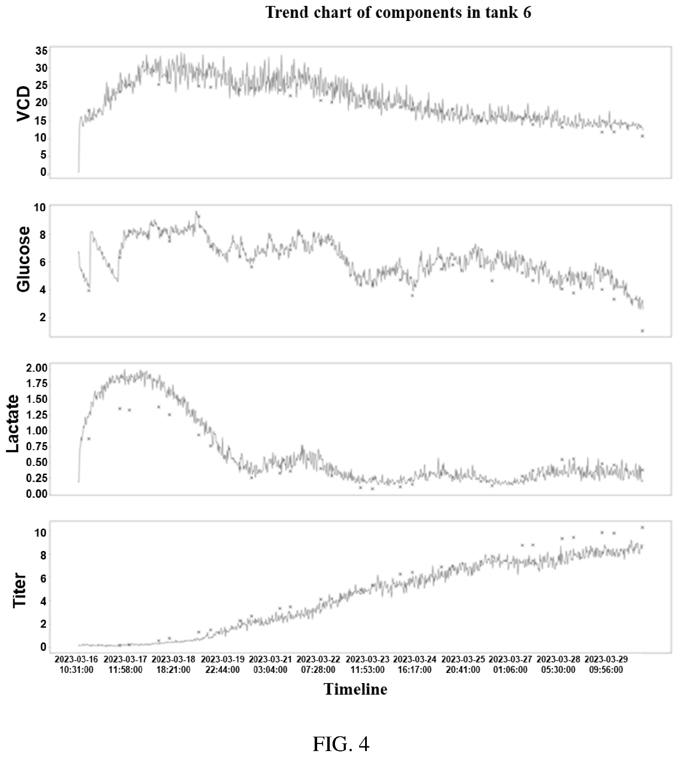

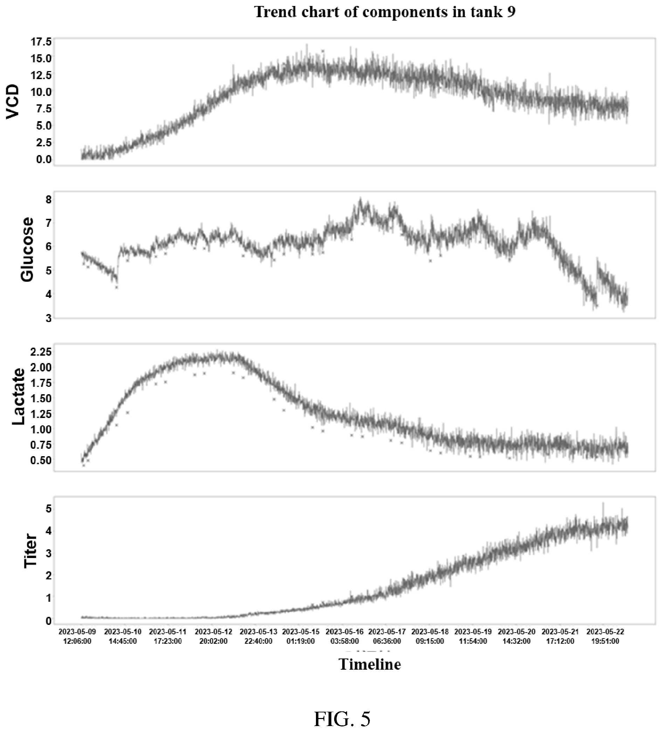

By comparing the performance of the RAFORMER-multimodal model with the performances of the two traditional models, GBDT and PLSR, for the above-mentioned two bioreactors, it can be found that for all 17 test items, the RAFORMER-multimodal model improves the Pearson correlation coefficient by 0.34 and 0.19 on average relative to GBDT and PLSR models, respectively, for the reactor tank 6, and by 0.07 and 0.08 for the reactor tank 9. For typical parameter trend changes of the tanks 6 and 9, please refer to FIG. 4 and FIG. 5 (the trend line represents the model predicted values, and the cross marks represent the actual values obtained from offline detection).

At step S230, preset-length spectral data of a detection-target cell culture solution at a target time point and a preset cell culture environmental parameter are obtained.

Specifically, the detection-target cell culture solution may be a cell culture solution in which concentrations of cell metabolites need to be analyzed in any biological reaction process. The target time point may be any time point in the biological reaction process of the detection-target cell culture solution.

The preset-length spectral data and the preset cell culture environmental parameter may be model input data that has undergone data preprocessing and has the same input paradigm as in the above-mentioned model training process.

In step S240, the spectral data and the preset cell culture environmental parameter are input into the target concentration prediction model to obtain a corresponding metabolite concentration prediction result of the detection-target cell culture solution at the target time point.

The trained target concentration prediction model can be directly used in the same application scenario to predict the concentration of the cell metabolite in the cell culture solution in any bioreactor that requires concentration prediction.

In the technical solution of this embodiment, the spectral sample data of the concentrations of different components of the cell culture solution at different time points, the corresponding preset cell culture environmental parameter sample data, and the offline concentration detected values are acquired, and the preset initial concentration prediction model is trained by taking the spectral sample data and the preset cell culture environmental parameter sample data as the model training input data and taking the offline concentration detected values at the corresponding time points as the model training label data, so that the target concentration prediction model is obtained. The target concentration prediction model includes a plurality of data feature extraction modules configured for extraction of data feature along different dimensions. Further, the trained model is applied. The preset-length spectral data of the detection-target cell culture solution at the target time point and the preset cell culture environmental parameter are acquired, and the spectral data and the preset cell culture environmental parameter are input into the target concentration prediction model to obtain the corresponding metabolite concentration prediction result of the detection-target cell culture solution at the target time point. The technical solution of the embodiment of the present disclosure solves the problem of inaccurate prediction results of existing cell culture solution concentration analysis models, and can perform data feature analysis based on multimodal data of the cell culture solution under test to obtain more accurate concentration prediction results fo the cell culture solution. In particular, by analyzing the preset-length spectral data of the detection-target cell culture solution at the target time point based on the self-attention mechanism, deeper-level data features can be captured to reflect the final prediction results.

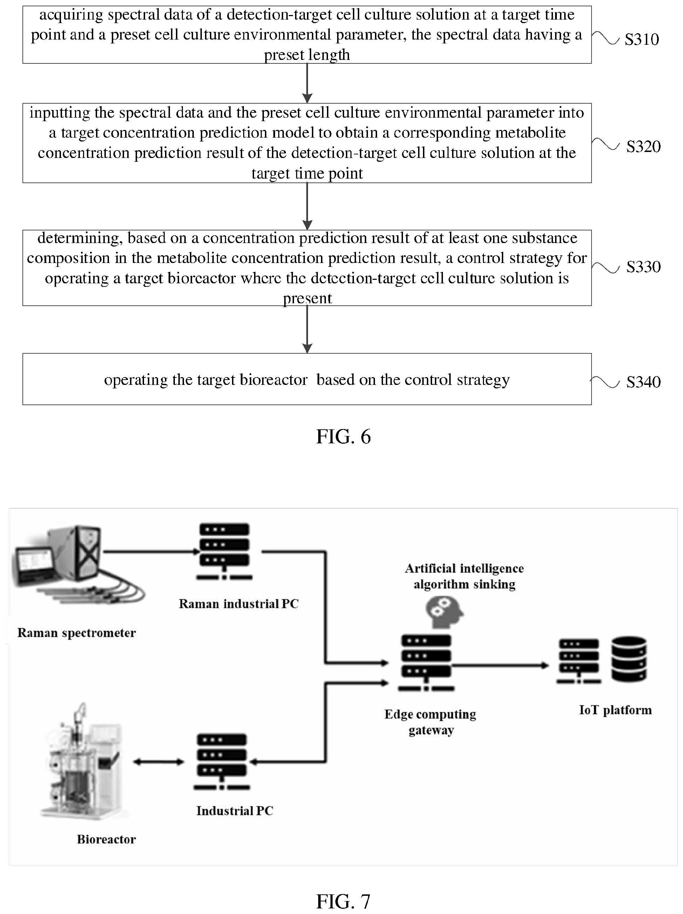

FIG. 6 is a flowchart of a method for determining a concentration of a metabolite in a cell culture solution according to some embodiments of the present disclosure. This embodiment has the same inventive concept as the methods for determining a concentration of a metabolite in a cell culture solution in the above embodiments, and further illustrates an application process of the concentration prediction result of the cell culture solution, especially the scenario of operating a bioreactor based on the prediction result. This method may be performed by an apparatus for determining a concentration of a metabolite in a cell culture solution, which may be implemented by software and/or hardware and integrated into a computer device with application development functions.

As shown in FIG. 6, the method for determining the concentration of the metabolite in the cell culture solution in this embodiment includes the following steps S310 to S340.

At step S310, preset-length spectral data of a detection-target cell culture solution at a target time point and a preset cell culture environmental parameter are obtained.

At step S320, the spectral data and the preset cell culture environmental parameter are input into a target concentration prediction model to obtain a corresponding metabolite concentration prediction result of the detection-target cell culture solution at the target time point.

Specifically, the target concentration prediction model may include plurality of data feature extraction modules, which are respectively configured to perform feature extraction on the spectral data and the preset cell culture environmental parameter along different feature analysis dimensions. In some embodiments, the plurality of data feature extraction modules include a data feature extraction module configured to extract, from the spectral data, a spectral feature between spectral data of different frequency bands through a self-attention mechanism. In some embodiments, multimodal higher-order features may then be fused to obtain the corresponding metabolite concentration prediction result of the detection-target cell culture solution at the target time point.

At step S330, a control strategy for operating a target bioreactor where the detection-target cell culture solution is present is determined based on a concentration prediction result of at least one substance composition in the metabolite concentration prediction result .

For example, the metabolite concentration prediction result may include information such as carbon dioxide concentration, concentrations of various nutrients, and cell concentration. Such concentration information may also be used to determine information such as osmotic pressure, temperature, and aeration/agitation state. When adjustment parameter indicators corresponding to various concentration values do not meet a standard condition, an adjustment may be made to generate a control strategy.

In some examples, the control strategy for the bioreactor may include a culture environment control strategy and a supplementing strategy, and the like. For example, the culture environment control strategy may include a carbon dioxide partial pressure strategy and a cooling strategy, and the like.

For example, in cell culture, carbon dioxide is not only a metabolic product of cells and an essential component for cell growth, but it is also related to maintaining the pH level of the culture solution. During cell culture, as a release amount of carbon dioxide increases, the culture solution becomes more acidic, and an alkaline solution is added or aeration is increased to maintain the pH level of the culture solution. Conversely, the culture solution will become alkaline, in which case carbon dioxide may be actively introduced to maintain the pH level. By using parameters such as carbon dioxide concentration and pH level in the metabolite concentration prediction result, the carbon dioxide concentration may be automatically increased or decreased according to the corresponding strategy, making the adjustment more real-time and more accurate.

For another example, during cell culture, once the viable cell density reaches a certain level, actively lowering the culture temperature can effectively maintain cell viability and increase the yield of the target protein. The temperature may be adjusted in a timely manner based on parameters such as cell density in the metabolite concentration prediction result.

In addition, during cell culture, it is necessary to supplement corresponding nutrients according to the rate at which the cells consume nutrients. Since there is no real-time feedback of offline detection data, the number of supplementing, the supplementing ratio, and the glucose concentration during the cell culture cycle in the supplementing strategy can only be set based on historical data and the experience of the experimenters. After the operators calculate the supplementing parameters according to the empirical formula, they manually add the supplementing materials into the bioreactor using a scale and a peristaltic pump. Based on the real-time metabolite concentration prediction result in this embodiment, timely supplementing can be carried out according to the preset supplementing rules, thereby improving the success rate of cell culture.

At step S340, the target bioreactor is operated based on the control strategy.

By synchronizing and implementing the control strategy determined in the above step in the target bioreactor, a corresponding control objective can be achieved, thereby ensuring the smooth progress of the biological reaction. For details, please refer to the process shown in FIG. 3. After performing the multi-test-item component prediction, a corresponding control strategy may be generated based on the prediction result, and an instruction corresponding to the control strategy is then sent to the target bioreactor for feedback control. At the same time, the cell culture solution in the bioreactor may be sampled and detected to increase the historical data in the database.

In some instances, Raman spectral data may be transmitted in real time via an Internet of Things (IoT) platform to a computer device (which may be an edge computing gateway device) configured with a self-attention encoder model (target concentration prediction model). The model predicts component indicators to generate state values for the respective indicators, and the bioreactor may be automatically controlled based on the state values, such as automatically controlling the glucose concentration in the bioreactor by feedback control. In some embodiments, the target concentration prediction model may be deployed on edge devices, and an Artificial Intelligence of Things (AIoT) device may be composed by integrating a Raman spectrometer, a Raman industrial personal computer (PC), an edge computing gateway, an IoT platform, an industrial PC, and a bioreactor, for monitoring and feedback control of the biological incubator, as shown in FIG. 7. The AIoT device can capture Raman spectral data in real time to predict concentrations of various components, combine the concentrations with other relevant parameters to calculate state values, and then use a corresponding automated control strategy to achieve automated control of cell culture in the bioreactor.

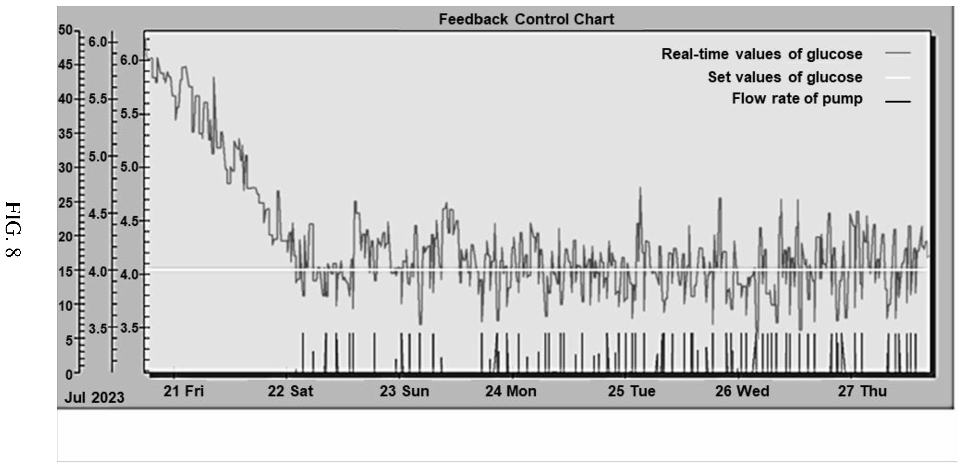

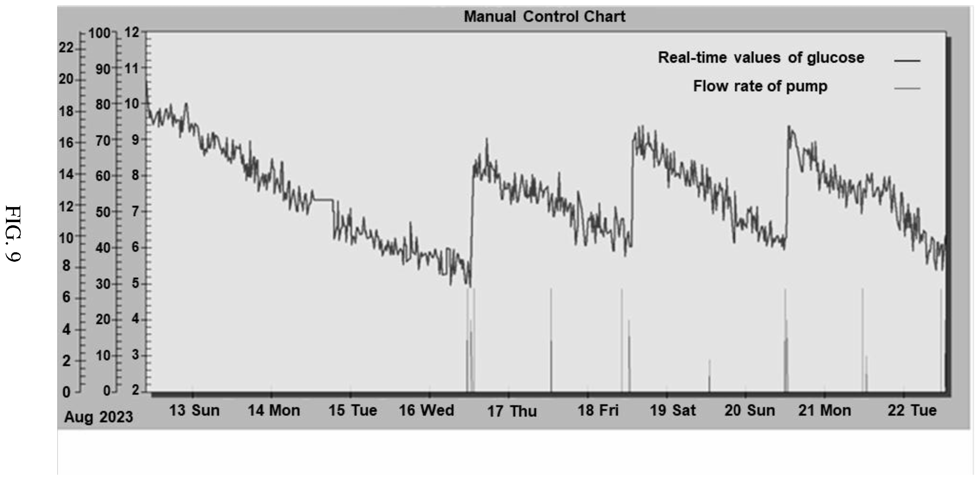

Furthermore, the comparison of glucose concentration changes under the biological reaction process control using the system described in FIG. 7 and under the biological reaction process manual control may be found in FIGS. 8 and 9. FIG. 8 shows a schematic diagram of the glucose concentration under the feedback control by the model matched with the peristaltic pump over an 8-day period. The glucose level under the feedback control by the model follows a set value of 4.0 g/L. FIG. 9 shows a schematic diagram of glucose level under the manual control following the experimental design range of 6-8g over an 11-day period. Over the 11 days, manually controlled supplementing is performed 7 times with a total of 270g, while automatic supplementing is performed multiple times in small amounts with a total of 216g. Therefore, when performing the real-time controlling, the amount of supplementing is reduced by about 20%, and the yield of the target protein does not decrease, but instead increases by approximately 4.03%. It can be determined that the control strategy for automated control of bioreactors based on the target concentration prediction model in this embodiment is superior.

In the technical solution of this embodiment, the preset-length spectral data of the detection-target cell culture solution at the target time point and the preset cell culture environmental parameter are acquired, and the spectral data and the preset cell culture environmental parameter are input into the target concentration prediction model to obtain the corresponding metabolite concentration prediction result of the detection-target cell culture solution at the target time point; the control strategy for operating the target bioreactor where the detection-target cell culture solution is present is determined based on the concentration prediction result of the at least one substance composition in the metabolite concentration prediction result; and the target bioreactor is operated based on the control strategy. The technical solution of the embodiment of the present disclosure solves the problem of inaccurate prediction results of existing cell culture solution concentration analysis models, and can perform data feature analysis based on multimodal data of the cell culture solution under test to obtain more accurate cell culture solution concentration prediction results. In particular, by analyzing the preset-length spectral data of the detection-target cell culture solution at the target time point based on the self-attention mechanism, deeper-level data features can be captured to reflect the final prediction result. This allows for real-time, more accurate, and automated adjustment of the bioreactor, ensuring the smooth progress of the biological reaction.

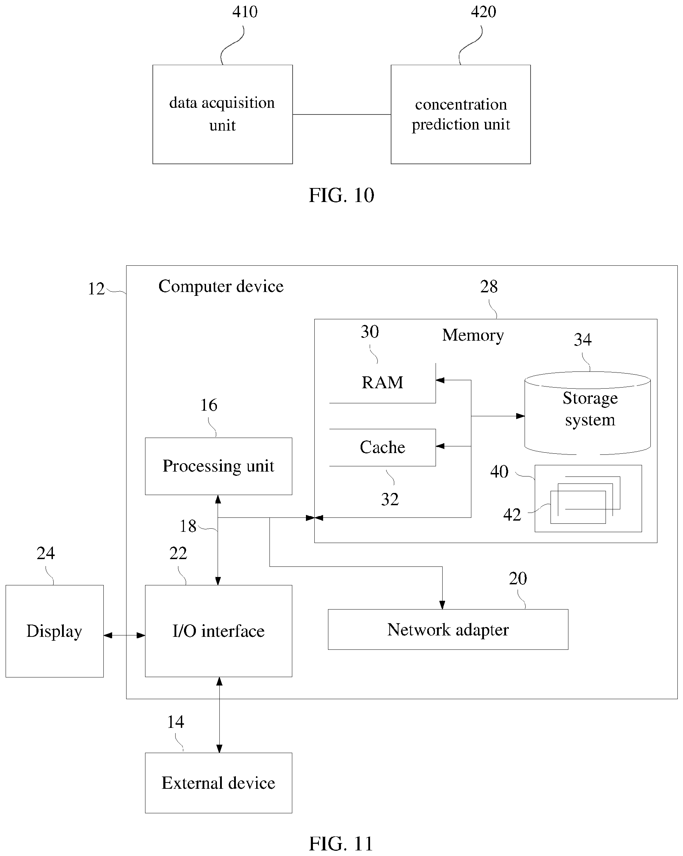

FIG. 10 is a schematic structural diagram of an apparatus for determining a concentration of a metabolite in a cell culture solution according to some embodiments of the present disclosure. This embodiment is applicable to biological experiment scenarios, especially cases where it is necessary to predict concentrations of cell metabolites during cell culture in bioreactors. The apparatus for determining the concentration of the metabolite in the cell culture solution may be implemented by software and/or hardware and integrated into a computer terminal device with application development functions.

As shown in FIG. 10, the apparatus for determining the concentration of the metabolite in the cell culture solution includes: a data acquisition unit 410 and a concentration prediction unit 420.

The data acquisition unit 410 is configured to acquire spectral data of a detection-target cell culture solution at a target time point and a preset cell culture environmental parameter, the spectral data having a preset length. The concentration prediction unit 420 is configured to input the spectral data and the preset cell culture environmental parameter into a target concentration prediction model to obtain a corresponding metabolite concentration prediction result of the detection-target cell culture solution at the target time point. The target concentration prediction model includes a plurality of data feature extraction modules, and the plurality of data feature extraction modules are respectively configured to perform feature extraction on the spectral data and the preset cell culture environmental parameter along different feature analysis dimensions.

In the technical solution of this embodiment, by acquiring the preset-length spectral data of the detection-target cell culture solution at the target time point and the preset cell culture environmental parameter, and by inputting the spectral data and the preset cell culture environmental parameter into the target concentration prediction model, the corresponding metabolite concentration prediction result of the detection-target cell culture solution at the target time point is obtained. The target concentration prediction model includes a plurality of data feature extraction modules, and the plurality of data feature extraction modules are respectively configured to perform feature extraction on the spectral data and the preset cell culture environmental parameter along different feature analysis dimensions. The technical solution of the embodiment of the present disclosure solves the problem of inaccurate prediction results of existing cell culture solution concentration analysis models, and can perform data feature analysis based on multimodal data of the cell culture solution under test to obtain more accurate cell culture solution concentration prediction results.

In some embodiments, the plurality of data feature extraction modules include a first data feature extraction module, a second data feature extraction module, and a third data feature extraction module;
the first data feature extraction module is configured to perform feature encoding analysis on the preset cell culture environmental parameter to obtain a target environmental parameter feature;
the second data feature extraction module is configured to extract, from the spectral data, a spectral feature between spectral data of different frequency bands; and
the third data feature extraction module is configured to extract at least one of following spectral features of the spectral data: a preset spectral statistics feature, a preset spectral feature, or a preset spectral peak analysis feature.

In some embodiments, the second data feature extraction module is configured to:
extract, from the spectral data, the spectral feature between the spectral data of different frequency bands by using a neural network module with a self-attention mechanism.

In some embodiments, the preset cell culture environmental parameter includes at least one of following parameters: a cell line parameter, a cell clone parameter, a culture process parameter, or parameters during cell culture.

In some embodiments, the target concentration prediction model further includes a feature fusion module and a concentration prediction module;
the feature fusion module is configured to perform feature fusion on data features extracted by the plurality of data feature extraction modules respectively to obtain a target fusion feature;
the concentration prediction module is configured to analyze the target fusion feature to obtain the metabolite concentration prediction result.

In some embodiments, the apparatus for determining the concentration of the metabolite in the cell culture solution further includes a model training module configured to obtain the target concentration prediction model by training, wherein a training process of the target concentration prediction model includes:
acquiring spectral sample data of concentrations of different components of a cell culture solution at different time points, corresponding preset cell culture environmental parameter sample data, and offline concentration detected values;
training a preset initial concentration prediction model by taking the spectral sample data and the preset cell culture environmental parameter sample data as model training input data and taking the offline concentration detected values at corresponding time points as model training label data, to obtain the target concentration prediction model.

In some embodiments, the apparatus for determining the concentration of the metabolite in the cell culture solution further includes a reactor operation strategy determination module configured to:
determine, based on a concentration prediction result of at least one substance composition in the metabolite concentration prediction result, a control strategy for operating a target bioreactor where the detection-target cell culture solution is present.

The apparatus for determining the concentration of the metabolite in the cell culture solution according to the embodiment of the present disclosure can execute the method for determining the concentration of the metabolite in the cell culture solution as provided in any embodiment of the present disclosure, and has the corresponding functional modules for executing the method and beneficial effects of the method.

FIG. 11 is a schematic structural diagram of a computer device according to some embodiments of the present disclosure. FIG. 11 shows a block diagram of an exemplary computer device 12 suitable for implementing embodiments of the present disclosure. The computer device 12 shown in FIG. 11 is merely an example and should not impose any limitation on the functionality and scope of use of the embodiments of the present disclosure. The computer device 12 may be any terminal device with computing capabilities, such as intelligent controllers, servers, mobile phones, and other terminal devices.

As shown in FIG. 11, the computer device 12 is represented in the form of a general-purpose computing device. Components of the computer device 12 may include, but are not limited to: one or more processors or processing units 16, a system memory 28, and a bus 18 connecting different system components (including the system memory 28 and the processing unit 16).

The bus 18 represents one or more of several bus architectures, including a memory bus or memory controller, a peripheral bus, a graphics acceleration port, a processor, or a local bus using any of various bus architectures. For example, these architectures include, but are not limited to, the Industrial Standard Architecture (ISA) bus, the Micro-Channel Architecture (MAC) bus, the Enhanced ISA bus, the Video Electronics Standards Association (VESA) local bus, and the Peripheral Component Interconnect (PCI) bus.

The computer device 12 typically includes various computer system readable media. These media may be any available media that can be accessed by the computer device 12, including volatile and non-volatile media, and removable and non-removable media.

The system memory 28 may include computer system readable media in the form of volatile memory, such as a random access memory (RAM) 30 and/or a cache memory 32. The computer device 12 may further include other removable/non-removable, volatile/non-volatile computer system storage media. By way of example only, a storage system 34 may be used to read from and write to non-removable, non-volatile magnetic media (not shown in FIG. 11, commonly referred to as "hard disk drives"). Although not shown in FIG. 11, a disk drive for reading from and writing to removable non-volatile disks (e.g., "floppy disks") and an optical disc drive for reading from and writing to removable non-volatile optical discs (e.g., Compact Disc Read-Only Memory (CD-ROM), Digital Versatile Disc Read-Only Memory (DVD-ROM), or other optical media) may be provided. In these cases, each driver may be connected to the bus 18 via one or more data media interfaces. The system memory 28 may include at least one program product having a set (e.g., at least one) of program modules configured to perform the functions of the embodiments of the present disclosure.

The program/utility 40 having a set (at least one) of program modules 42 may be stored, for example, in the system memory 28. Such program modules 42 include, but are not limited to, an operating system, one or more application programs, other program modules, and program data. Each of or some combination of these examples may include an implementation of a network environment. The program module 42 typically performs the functions and/or methods described in the embodiments of the present disclosure.

The computer device 12 may also communicate with one or more external devices 14 (e.g., keyboard, pointing device, display 24, etc.), and may also communicate with one or more devices that enable a user to interact with the computer device 12, and/or communicate with any device that enables the computer device 12 to communicate with one or more other computing devices (e.g., network card, modem, etc.). This communication may be performed via an input/output (I/O) interface 22. Furthermore, the computer device 12 may also communicate with one or more networks (such as a local area network (LAN), a wide area network (WAN), and/or a public network, such as the Internet) via a network adapter 20. As shown in the figure, the network adapter 20 communicates with other modules of computer device 12 via the bus 18. It should be understood that, although not shown in FIG. 11, the computer device 12 may be used in conjunction with other hardware and/or software modules, including but not limited to: microcode, device drivers, redundant processing units, external disk drive arrays, Redundant Array of Independent Disk (RAID) systems, tape drives, and data backup storage systems, etc.

The processing unit 16 executes various functional applications and data processing by running programs stored in the system memory 28, such as implementing the method for determining the concentration of the metabolite in the cell culture solution according to embodiments of the present disclosure, the method including:
acquiring spectral data of a detection-target cell culture solution at a target time point and a preset cell culture environmental parameter, the spectral data having a preset length;
inputting the spectral data and the preset cell culture environmental parameter into a target concentration prediction model to obtain a corresponding metabolite concentration prediction result of the detection-target cell culture solution at the target time point,
wherein the target concentration prediction model includes a plurality of data feature extraction modules, and the plurality of data feature extraction modules are respectively configured to perform feature extraction on the spectral data and the preset cell culture environmental parameter along different feature analysis dimensions.

The present disclosure further provides a non-transitory computer-readable storage medium storing a computer program thereon. When executed by a processor, the program implements the method for determining the concentration of the metabolite in the cell culture solution as provided in any embodiment of the present disclosure, the method including:
acquiring spectral data of a detection-target cell culture solution at a target time point and a preset cell culture environmental parameter, the spectral data having a preset length;
inputting the spectral data and the preset cell culture environmental parameter into a target concentration prediction model to obtain a corresponding metabolite concentration prediction result of the detection-target cell culture solution at the target time point,
wherein the target concentration prediction model includes a plurality of data feature extraction modules, and the plurality of data feature extraction modules are respectively configured to perform feature extraction on the spectral data and the preset cell culture environmental parameter along different feature analysis dimensions.

The computer storage medium of the embodiments of the present disclosure may employ any combination of one or more computer-readable media. The computer-readable medium may be a computer-readable signal medium or a computer-readable storage medium. The computer-readable storage medium may be, for example, but not limited to, an electrical, magnetic, optical, electromagnetic, infrared, or semiconductor system, apparatus, or device, or any combination thereof. More specific examples of computer-readable storage media (a non-exhaustive list) include: electrical connections having one or more wires, portable computer disks, hard disks, random access memory (RAM), read-only memory (ROM), erasable programmable read-only memory (EPROM or flash memory), optical fiber, portable compact disk read-only memory (CD-ROM), optical storage devices, magnetic storage devices, or any suitable combination of the foregoing. In this document, the computer-readable storage medium can be any tangible medium that contains or stores a program, which can be used by or in connection with an instruction execution system, apparatus, or device.

The computer-readable signal medium may include a data signal propagated in a baseband or as part of a carrier wave, which carries computer-readable program code. The transmitted data signal may take many forms, including but not limited to an electromagnetic signal, an optical signal, or any suitable combination thereof. The computer-readable signal medium may also be any computer-readable medium other than the computer-readable storage medium, which can send, propagate or transmit a program for use by or in connection with an instruction execution system, apparatus or device.

The program code contained on the computer-readable medium may be transmitted using any suitable medium, including but not limited to: wireless, wire, optical fiber, radio frequency (RF), etc., or any suitable combination thereof.

Computer program codes for performing the operations of the present disclosure may be written in one or more programming languages or a combination thereof, including object-oriented programming languages such as Java, Smalltalk, and C++, as well as conventional procedural programming languages such as the "C" language or similar programming languages. The program code may be executed entirely on the user's computer, partially on the user's computer, as a standalone software package, partially on the user's computer and partially on a remote computer, or entirely on a remote computer or server. In cases involving remote computers, the remote computer may be connected to the user's computer via any type of network, including a local area network (LAN) or a wide area network (WAN), or may be connected to an external computer (e.g., via the Internet through an Internet service provider).

Those skilled in the art should understand that the modules or steps of the present disclosure described above may be implemented using general-purpose computing devices, may be centralized on a single computing device or distributed across a network composed of multiple computing devices, may optionally be implemented using computer device-executable program code (so as to be stored in a storage device for execution by a computing device), or may be fabricated into separate integrated circuit modules, or multiple modules or steps thereof may be fabricated into a single integrated circuit module. Thus, the present disclosure is not limited to any particular combination of hardware and software.

Note that the above are merely preferred embodiments and technical principles of the present disclosure. Those skilled in the art will understand that the present disclosure is not limited to the specific embodiments described herein, and that various obvious changes, readjustments, and substitutions can be made by those skilled in the art without departing from the scope of protection of the present disclosure. Therefore, although the present disclosure has been described in detail through the above embodiments, the present disclosure is not limited to the above embodiments. More other equivalent embodiments may be included without departing from the concept of the present disclosure, and the scope of the present disclosure is determined by the scope of the appended claims.

## Claims

1. A method for determining a concentration of a metabolite in a cell culture solution, the method comprising:
acquiring spectral data of a detection-target cell culture solution at a target time point and a preset cell culture environmental parameter, the spectral data having a preset length; and
inputting the spectral data and the preset cell culture environmental parameter into a target concentration prediction model to obtain a corresponding metabolite concentration prediction result of the detection-target cell culture solution at the target time point,
wherein the target concentration prediction model comprises a plurality of data feature extraction modules, and the plurality of data feature extraction modules are respectively configured to perform feature extraction on the spectral data and the preset cell culture environmental parameter along different feature analysis dimensions.

2. The method of claim 1, wherein the plurality of data feature extraction modules comprise a first data feature extraction module, a second data feature extraction module, and a third data feature extraction module;
wherein the first data feature extraction module is configured to perform feature encoding analysis on the preset cell culture environmental parameter to obtain a target environmental parameter feature;
the second data feature extraction module is configured to extract, from the spectral data, a spectral feature between spectral data of different frequency bands; and
the third data feature extraction module is configured to extract at least one of following spectral features of the spectral data: a preset spectral statistics feature, a preset spectral feature, or a preset spectral peak analysis feature.

3. The method of claim 2, wherein the second data feature extraction module is configured to:
extract, from the spectral data, the spectral feature between the spectral data of different frequency bands by using a neural network module with a self-attention mechanism.

4. The method of claim 1, wherein the preset cell culture environmental parameter comprises at least one of following parameters: a cell line parameter, a cell clone parameter, a culture process parameter, or parameters during cell culture.

5. The method of any one of claims 1 to 4, wherein the target concentration prediction model further comprises a feature fusion module and a concentration prediction module;
wherein the feature fusion module is configured to perform feature fusion on data features extracted by the plurality of data feature extraction modules respectively to obtain a target fusion feature; and
the concentration prediction module is configured to analyze the target fusion feature to obtain the metabolite concentration prediction result.

6. The method of any one of claims 1 to 4, wherein a training process of the target concentration prediction model comprises:
acquiring spectral sample data of concentrations of different components of a cell culture solution at different time points, corresponding preset cell culture environmental parameter sample data, and offline concentration detected values; and
training a preset initial concentration prediction model by taking the spectral sample data and the preset cell culture environmental parameter sample data as model training input data and taking the offline concentration detected values at corresponding time points as model training label data, to obtain the target concentration prediction model.

7. The method of any one of claims 1 to 4, further comprising:
determining, based on a concentration prediction result of at least one substance composition in the metabolite concentration prediction result, a control strategy for operating a target bioreactor where the detection-target cell culture solution is present.

8. An apparatus for determining a concentration of a metabolite in a cell culture solution, the apparatus comprising:
a data acquisition unit configured to acquire spectral data of a detection-target cell culture solution at a target time point and a preset cell culture environmental parameter, the spectral data having a preset length; and
a concentration prediction unit configured to input the spectral data and the preset cell culture environmental parameter into a target concentration prediction model to obtain a corresponding metabolite concentration prediction result of the detection-target cell culture solution at the target time point,
wherein the target concentration prediction model comprises a plurality of data feature extraction modules, and the plurality of data feature extraction modules are respectively configured to perform feature extraction on the spectral data and the preset cell culture environmental parameter along different feature analysis dimensions.

9. A computer device, wherein the computer device comprises:
one or more processors; and
a memory configured to store one or more programs,
wherein the one or more programs, when executed by the one or more processors, cause the one or more processors to implement the method for determining the concentration of the metabolite in the cell culture solution of any one of claims 1 to 7.

10. A non-transitory computer-readable storage medium having a computer program stored thereon which, when executed by a processor, implements the method for determining the concentration of the metabolite in the cell culture solution of any one of claims 1 to 7.
